# EUROPEAN PATENT SPECIFICATION

(11) **EP 3 871 594 B1**
(45) Date of publication and mention of the grant of the patent: **29.07.2026**
(21) Application number: 19876503.4
(22) Date of filing: 25.10.2019
(51) Int. Cl.: A61B 5/00, A61B 5/283, A61B 5/347, A61B 5/346, A61B 5/361, A61B 5/363, A61B 5/367

(54) **APPARATUS FOR ANALYZING ELECTROCARDIO SIGNAL, AND SIGNAL RECORDER AND THREE-DIMENSIONAL MAPPING SYSTEM**
VORRICHTUNG ZUR ANALYSE VON ELEKTROKARDIOSIGNALEN SOWIE SIGNALAUFZEICHNUNGSGERÄT UND DREIDIMENSIONALES ABBILDUNGSSYSTEM
APPAREIL D'ANALYSE DE SIGNAL ÉLECTROCARDIOGRAPHIQUE, ET ENREGISTREUR DE SIGNAL ET SYSTÈME DE CARTOGRAPHIE TRIDIMENSIONNELLE

(30) Priority: 25.10.2018 CN 201811250703
(43) Date of publication of application: 01.09.2021
(73) Proprietor: Shanghai Microport Ep Medtech Co., Ltd., Shanghai 201318 (CN)
(72) Inventor: SUN, Yiyong, Shanghai 201318 (CN); HUANG, Hao, Shanghai 201318 (CN); WANG, Xinyi, Shanghai 201318 (CN); ZHANG, Qingchun, Shanghai 201318 (CN); CAO, Xianfeng, Shanghai 201318 (CN); SHEN, Liuping, Shanghai 201318 (CN)
(74) Representative: Marks & Clerk LLP
(86) International application number: PCT/CN2019/113424
(87) International publication number: WO 2020/083392

(56) References cited:
- CN-A- 101 292 870
- CN-A- 101 836 862
- CN-A- 101 933 803
- CN-A- 104 840 196
- CN-A- 106 061 374
- JP-A- S6 156 635
- US-A1- 2004 059 237
- US-A1- 2004 127 805
- US-A1- 2004 127 805

## Description

### TECHNICAL FIELD

The present disclosure relates to the field of medical technology, particularly to an apparatus for analyzing an electrocardiography (ECG) signal, an electrophysiological signal recorder, a three-dimensional mapping system, and a computer device.

### BACKGROUND

At present, for complex arrhythmia diseases, such as atrial fibrillation, nonspecific supraventricular tachycardia, etc., since there are usually multiple focal points or multiple abnormal propagation paths, which cause confusion in intracardiac signals, it is difficult for doctors to determine the focal points. The analysis provided by the existing computer-aided focal lesion determination method and system is not enough to solve the above problem, which brings great challenges to the treatment of the disease.

The document US 2004/059237 A1 discloses that analyzes surface electrocardiographic and intracardiac signals to identify and separate electrical activity corresponding to distinct but superimposed events in the heart. Assesses the spatial phase, temporal phase, rate, spectrum and reproducibility of each event to determine uniformity of activation in all spatial dimensions. Uses numerical indices derived from these analyses to diagnose arrhythmias. Uses these indices to determine the location of an arrhythmia circuit, and to direct the movement of an electrode catheter to this location for ablation or permanent catheter positioning. Subsequently, uses these indices to determine whether ablation has successfully eliminated the circuit. Uses variability in these indices from the surface electrocardiogram to indicate subtle beat-to-beat fluctuations which reflect the tendency towards atrial and ventricular arrhythmias.

### SUMMARY

Various exemplary embodiments in the present disclosure provide an apparatus for analyzing an ECG signal, an electrophysiological signal recorder, a three-dimensional mapping system, and a computer device.

In an aspect of the present disclosure, an apparatus for analyzing an ECG signal is provided, including:
a first acquisition module, configured to acquire N first intracardiac electrical signals in a region of interest or at a reference point/in a reference region to obtain N first intracardiac waveforms, wherein N is an integer greater than or equal to 1;
a first generation module, configured to generate a first comparison signal spectrogram according to the N first intracardiac waveforms;
a second acquisition module, configured to acquire M second intracardiac electrical signals at the point of interest to acquire M second intracardiac waveforms, wherein M is an integer greater than or equal to 1;
a second generation module, configured to generate a second comparison signal spectrogram according to the M second intracardiac waveforms;
a first analysis module, configured to perform a comparative analysis on the first comparison signal spectrogram and the second comparison signal spectrogram, and generate a comparative analysis result; and
an output module, configured to output prompt information indicating whether the point of interest is a focal point according to the comparative analysis result; the first generation module further includes:
a first processing unit, configured to generate a first single-signal spectrogram of each first intracardiac waveform to obtain N first single-signal spectrograms; and
a second processing unit, configured to combine the N first single-signal spectrograms to generate the first comparison signal spectrogram, or select one first single-signal spectrogram from the N first single-signal spectrograms as the first comparison signal spectrogram.

In another aspect of the present disclosure, an electrophysiological signal recorder is provided, including the aforementioned apparatus for analyzing the ECG signal.

In another aspect of the present disclosure, a three-dimensional mapping system is further provided, including the aforementioned apparatus for analyzing the ECG signal, and a display module configured to display a three-dimensional model of a heart; the output module is configured to output the prompt information to the display module which displays the prompt information on the three-dimensional model of the heart.

In the aforementioned apparatus for analyzing the ECG signal, electrophysiological signal recorder, three-dimensional mapping system, and the computer device, the N first intracardiac electrical signals in the region of interest or at the reference point/in the reference region are acquired, and then the N first intracardiac waveforms are obtained, and the first comparison signal spectrogram is generated according to the N first intracardiac waveforms, and the M second intracardiac electrical signals at the point of interest are acquired, and then the M second intracardiac waveforms are obtained, and a second comparison signal spectrogram is generated according to the M second intracardiac waveforms, and a comparative analysis is performed on the first comparison signal spectrogram and the second comparison signal spectrogram and a comparative analysis result is generated, prompt information indicating whether the point of interest is a focal point is outputted according to the comparative analysis result. Thus, by using a computer system to perform spectrogram analysis on intracardiac electrical signals, and by comparing and determining the spectrograms of the region of interest or the reference point/region and the point of interest, the determination of the focal point can be more accurate, thereby providing the doctors with more effective information, such that the cure rate of complex arrhythmia diseases is effectively improved.

### BRIEF DESCRIPTION OF THE DRAWINGS

FIG 1 is an application environment diagram of a method for analyzing an ECG signal according to an embodiment of the present disclosure;
FIG 2 is a flow chart showing a method for analyzing an ECG signal according to an embodiment of the present disclosure;
FIG 3 is a flow chart showing an acquisition of a first comparison signal spectrogram according to an embodiment of the present disclosure;
FIG 4 is a flow chart showing an acquisition of a first single-signal spectrogram according to an embodiment of the present disclosure;
FIG 5 is a schematic diagram of pre-processing a first intracardiac waveform according to an embodiment of the present disclosure;
FIG 6 is a schematic diagram of smoothing an original signal spectrogram according to an embodiment of the present disclosure;
FIG 7 is a flow chart of generating a first comparison signal spectrogram by curve accumulation according to an embodiment of the present disclosure;
FIG 8 is a schematic diagram of generating a first comparison signal spectrogram by curve accumulation according to an embodiment of the present disclosure;
FIG 9 is a flow chart of generating a first comparison signal spectrogram by a probabilistic method according to an embodiment of the present disclosure;
FIG 10 is a flow chart of acquiring a first comparison signal spectrogram according to another embodiment of the present disclosure;
FIG 11 is a schematic diagram of a first comparison signal spectrogram according to an embodiment of the present disclosure;
FIG 12 is a flow chart of determining a point of interest according to an embodiment of the present disclosure;
FIG 13 is a flow chart of determining a point of interest according to another embodiment of the present disclosure;
FIG 14 is a flow chart of determining diagnosis and treatment effects according to an embodiment of the present disclosure;
FIG 15 is a schematic diagram of analyzing an ECG signal for atrial fibrillation according to an embodiment of the present disclosure;
FIG. 16 is a structure block diagram of an apparatus for analyzing an ECG signal according to an embodiment of the present disclosure;
FIG. 17 is a structure block diagram of an apparatus for analyzing an ECG signal according to another embodiment of the present disclosure;
FIG. 18 is a structure block diagram of an apparatus for analyzing an ECG signal according to another embodiment of the present disclosure;
FIG. 19 is a structure block diagram of an apparatus for analyzing an ECG signal according to another embodiment of the present disclosure;
FIG. 20 is a structure block diagram of an electrophysiological signal recorder according to an embodiment of the present disclosure;
FIG. 21 is a structure block diagram of a three-dimensional mapping system according to an embodiment of the present disclosure;
FIG. 22 is a schematic diagram of a three-dimensional heart display in a three-dimensional mapping system according to another embodiment of the present disclosure.

### DETAILED DESCRIPTION

In order to make the purpose, technical solution and advantages of the present disclosure clearer, the present disclosure will be described in further detail in conjunction with the accompanying drawings and embodiments. It should be understood that the specific embodiments described herein are only used for explaining the present disclosure, rather than limiting the present disclosure.

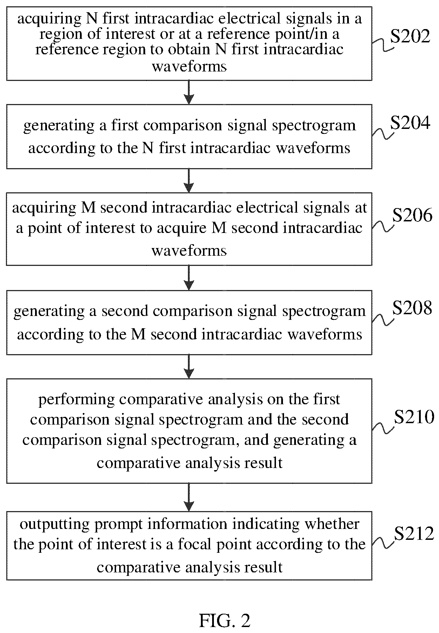

It should be noted that the following method embodiments described with reference to FIGS. 2-4, 7, 9, 10, and 12-14 are not according to the invention and are present for illustration purposes only.

In the diagnosis and treatment of arrhythmia disease known to the inventors, the doctor will place a mapping catheter connected to an electrophysiological signal recorder or a three-dimensional mapping system in a region of interest. At this time, the electrophysiological signal recorder or the three-dimensional mapping system can obtain an intracardiac electrical signal in the region of interest through the mapping catheter, and present the intracardiac electrical signal to the doctor, so that the doctor can determine a focal point or an abnormal propagation path of a disease based on the intracardiac electrical signal. However, for complex arrhythmia diseases, there are usually multiple focal points or multiple abnormal propagation paths, in other words, the complex arrhythmia diseases are a result of multiple focal points or multiple abnormal propagation paths acting together. The present technology only focuses on determining the focal point or the abnormal propagation paths of the disease by acquiring the intracardiac electrical signal of one single point or one single region, which often leads to inaccurate and incomplete determination result. For example, the ablation region is wide but inaccurate, which increases patient's pain and reduces the success rate of an operation. The inventors of the present disclosure, through in-depth analysis on the intracardiac electrical signals, found that a technique is needed to identify and distinguish these abnormalities, and treat these abnormalities differently, in order to provide the doctor with more information. On the other hand, the inventors have further creatively found that regular data can be obtained from the complex intracardiac electrical signals by performing frequency domain processing on these intracardiac electrical signals and comparing the frequency domains or frequencies of different regions or different points, and then the electrophysiological behavior of the heart can be more accurately determined based on these data, accordingly the accuracy and comprehensiveness of the determination of abnormal focal points or abnormal propagation paths can be further improved. Based on this, various exemplary embodiments of the present disclosure provide a method and an apparatus for analyzing an ECG signal, an electrophysiological signal recorder, a three-dimensional mapping system, and a computer device.

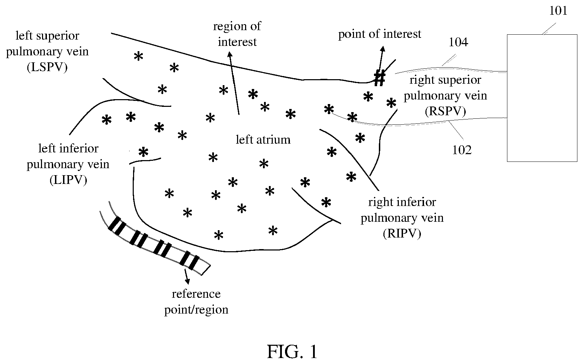

A method for analyzing an ECG signal provided by an embodiment of the present disclosure can be applied in the application environment shown in FIG 1. The controller 101 can be connected to an external signal collection device, such as an electrophysiological mapping catheter. The intracardiac electrical signal is collected through the mapping catheter, and then the controller 101 acquires the intracardiac electrical signal through the mapping catheter. Specifically, the controller 101 can acquire N first intracardiac electrical signals in a region of interest through a first mapping catheter 102 (* in the figures represents a collection point), so that corresponding N first intracardiac waveforms are obtained according to the N first intracardiac electrical signals, and a first comparison signal spectrogram is generated according to the N first intracardiac waveforms, or the N first intracardiac electrical signals are acquired in a reference point/region through the first mapping catheter 102 (a black line in the figures represents the collection point), thereby obtaining the N first intracardiac waveforms, and generating the first comparison signal spectrogram according to the N first intracardiac waveforms. The controller 101 can also acquire M second intracardiac electrical signals of a point of interest through a second mapping catheter 104, thereby obtaining corresponding M second intracardiac waveforms according to the M second intracardiac electrical signals, and generating a second comparison signal spectrogram according to the M second intracardiac waveforms. Finally, the controller 101 compares and analyzes the first comparison signal spectrogram with the second comparison signal spectrogram to generate a comparative analysis result, and outputs prompt information indicating whether the point of interest is a focal point according to the comparative analysis result. Here, N and M are integers greater than or equal to 1, and N may be the same as or different form M.

In an embodiment, as shown in FIG 2, a method for analyzing an ECG signal is provided. The method applied to the controller of the FIG 1 is taken as an example for description, including the following steps.

Step 202: N first intracardiac electrical signals in a region of interest or a reference point/region are acquired, to obtain N first intracardiac waveforms according to the N first intracardiac electrical signals; N is an integer greater than or equal to 1.

For different arrhythmia diseases, the region of interest is also different. For example, the region of interest of the atrial fibrillation is generally the left atrium; the region of interest of ventricular premature beats is generally the right ventricle or left ventricle; and the region of interest of the supraventricular tachycardia is generally the right atrium. The mapping catheter is generally fixedly provided in a reference region a position of which does not change during the entire treatment process. The reference point can be a point in the reference region. For example, the reference region of the atrial fibrillation is coronary sinus, and the reference point thereof is a point in the coronary sinus. The reference region of the supraventricular tachycardia is the right atrium, and the reference point thereof is a point in the right atrium. A reference region of atrial flutter is the left atrium/right atrium, and the reference point thereof is a point in the left atrium/right atrium. Specifically, the doctor can place a coronary sinus catheter for the atrial fibrillation, a right atrial mapping catheter for the supraventricular tachycardia, or an atrial flutter large ring mapping catheter for the atrial flutter, according to the specific situation of the patient, to acquire the intracardiac electrical signal at the reference point/in the reference region.

When the first intracardiac electrical signal in the region of interest or at the reference point/in the reference region is acquired, one or more first intracardiac electrical signals can be acquired, thereby obtaining one or more first intracardiac waveforms. The specific number can be selected according to the size of the region of interest or the reference point/region. The more the first intracardiac electrical signal, the better overall intracardiac situation in the region of interest or at the reference point/in the reference region can be acquired. For example, since the region of interest for the atrial fibrillation is generally in the left atrium, in order to comprehend the features of the entire left atrium as a whole, multiple collection points can be set in the left atrium (multiple collection points as shown in FIG. 1). Then, the first intracardiac electrical signal is collected at each collection point to obtain an intracardiac waveform which represents the first intracardiac waveform. For example, when there are fifty collection points, then fifty first intracardiac waveforms can be obtained.

Specifically, the intracardiac electrical signal can be collected through the mapping catheter. As the development of the electrophysiological mapping technology, the mapping catheter has been developed to have a configuration with multiple electrodes, so that it can collect the intracardiac electrical signals from multiple positions at the same time. For example, sixty-four intracardiac electrical signals can be collected at the same time, so that sixty-four intracardiac waveforms can be obtained in one collection. In such a way, when N intracardiac electrical signals need to be collected, N collections can be performed through a mapping catheter with one electrode, or [N/a] collections can be performed through a mapping catheter with a electrodes. Specific options can be performed according to the real-time requirements. For example, when it is needed to acquire the features of the entire left atrium, fifty to two hundred collections may be required if a mapping catheter with one electrode is adopted; and if a mapping catheter with 10 electrodes is adopted, only five to twenty collections may be required. For the latter, although the number of collections is less, the simultaneously processed loads are high, accordingly the real-time performance of data processing is relatively lower. In a preferred embodiment, a mapping catheter with four to ten electrodes is adopted to meet the requirement of the real-time performance while reducing the number of collections.

A first mapping catheter 102 with five electrodes is taken as an example, the doctor can place the first mapping catheter 102 at a certain position in the left atrium when performing one collection. In this case, the controller 101 can start collecting the intracardiac electrical signal at the certain position through the first mapping catheter 102, and stop collecting when collection time reaches a preset time, and thus one collection of the intracardiac electrical signal is completed. Since the number of electrodes of the mapping catheter 102 is five in this embodiment, after the collection is completed, five first intracardiac waveforms, that is, five timing waveforms of the intracardiac electrical signals from different collection points, can be acquired according to collected the intracardiac electrical signals. Then, the doctor can place the first mapping catheter 102 at another position in the left atrium. In this case, the controller 101 can perform a second collection through the first mapping catheter 102, and acquire another five first intracardiac waveforms according to the collected intracardiac electrical signals, and so on until all collections are completed.

The collection time is time to analyze a timing sequence of the intracardiac electrical signal, that is, a duration of the first intracardiac waveform, and a range thereof can be 1s to 10s, preferably 10s, that is, intracardiac electrical signals collected every 10s are taken as a unit to form a corresponding first intracardiac waveform for subsequent spectral analysis. In practical application, a default collection time such as 10s can be set in the controller 101, and an analysis window can also be set at the same time, so that the doctor can adjust the collection time through the analysis window to meet actual needs.

In addition, the intracardiac electrical signal collected by the mapping catheter can be a bipolar signal or a unipolar signal. Since the bipolar signal can effectively suppress an interference of a common-mode signal, the bipolar signal is generally preferred. This ensures that the collected intracardiac electrical signals are not interfered with environment noise, making the collected intracardiac waveforms more accurate.

Step 204: a first comparison signal spectrogram is generated according to the N first intracardiac waveforms.

Specifically, after the collection of the first intracardiac waveform in the region of interest or at the reference point/in the reference region is completed, frequency domain conversion, combination processing, etc., can be performed on the N first intracardiac waveforms collected to generate the first comparison signal spectrogram of the region of interest or the reference point/region.

Step 206: M second intracardiac electrical signals at a point of interest are acquired, and M corresponding second intracardiac waveforms are obtained according to the M second intracardiac electrical signals, M is an integer greater than or equal to 1.

The point of interest is generally a problem-prone part obtained through the analysis on the clinical history data in the disease treatment. For example, the point of interest of the atrial fibrillation can be preferably a point in the pulmonary vein, and the point of interest of the atrial flutter can be preferably a point in the tricuspid isthmus.

When the second intracardiac electrical signal at the point of interest is acquired, one or more second intracardiac electrical signals can be acquired, and accordingly the corresponding one or more second intracardiac waveforms are obtained. The specific number can be selected according to the size of the point of interest. Specifically, the atrial fibrillation is still taken as an example. After the first comparison signal spectrogram of the region of interest or the reference point/region is obtained, the doctor can place the mapping catheter 104 for the pulmonary vein. In this case, the controller 101 can start collecting the intracardiac electrical signal at the pulmonary vein through the second mapping catheter 104, and stop collecting when the collection time reaches the preset time, and thus one collection of the intracardiac electrical signal is completed. When the number of electrodes of the second mapping catheter 104 is one, one second intracardiac waveform can be obtained through one collection; when the number of electrodes of the second mapping catheter 104 is five, five second intracardiac waveforms can be obtained through one collection.

Step 208: a second comparison signal spectrogram is generated according to the M second intracardiac waveforms.

Specifically, after the collection of the second intracardiac waveform of the point of interest is completed, frequency domain conversion and combination processing can be performed on the M second intracardiac waveforms collected to generate the second comparison signal spectrogram of the point of interest.

Step 210: comparative analysis is performed on the first comparison signal spectrogram and the second comparison signal spectrogram, and a comparative analysis result is generated.

Step 212: prompt information indicating whether the point of interest is a focal point is output according to the comparative analysis result.

Specifically, after the first comparison signal spectrogram of the region of interest or the reference point/region and the second comparison signal spectrogram of the point of interest are obtained, the comparative analysis can be performed on the two spectrograms. For example, the comparative analysis is performed on an energy and a curve shape of the spectrograms to output the prompt information indicating whether the point of interest is the focal point. For example, the prompt information indicating whether the point of interest is the focal point can be output in the manner of text, image, or voice. The specific manner can be selected according to actual needs.

In this embodiment, the spectral analysis is performed on the intracardiac electrical signal and the comparative determination is performed on the spectrograms of the region of interest or the reference point/region and the point of interest, that is, the global and the local spectrums are compared, and the prompt information indicating whether the point of interest is a focal point is output based on the result of the comparative analysis, the problem of difficult disease determination caused by the confusion in the intracardiac signals can be effectively solved. In addition, compared to the determination result of the focal point provided only by the spectrograms of the point of interest, the accuracy is higher, thereby providing more favorable information for the treatment of the disease.

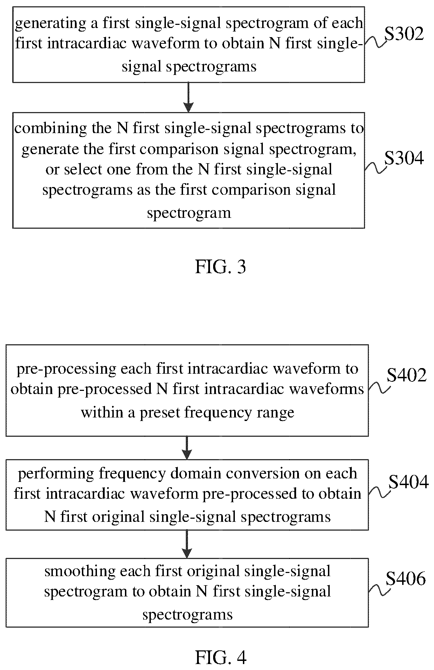

In an embodiment, as shown in FIG 3, the step of generating a first comparison signal spectrogram according to the N first intracardiac waveforms can further include steps S302 and S304.

Step 302: a first single-signal spectrogram of each first intracardiac waveform is generated to obtain N first single-signal spectrograms.

In an embodiment, the step of generating the first single-signal spectrogram of each first intracardiac waveform to obtain the N first single-signal spectrograms further includes: a frequency domain conversion is performed on each first intracardiac waveform to acquire the N first single-signal spectrograms. For example, Fast Fourier Transform or Wavelet Transform can be adopted to perform the frequency domain conversion on each first intracardiac waveform, to obtain the N first single-signal spectrograms.

Specifically, after the collection of the N-th first intracardiac electrical signal is completed, the N first intracardiac waveforms and N single-signal spectrograms corresponding to the N first intracardiac waveforms are generated uniformly, the N single-signal spectrograms represents the N first single-signal spectrograms (that is, a signal spectrogram corresponding to each first intracardiac waveform is referred to as the first single-signal spectrogram), or one first intracardiac electrical signal collected is processed after each collection is completed, to generate the first intracardiac waveform and the first single-signal spectrogram corresponding to the first intracardiac waveform. In a preferred embodiment, the latter is adopted, so that the problem of long analysis time caused by the centralized data processing can be effectively reduced, and the real-time performance of the signal spectrogram can be ensured.

For example, when the number of electrodes of the first mapping catheter 102 is five, after one collection is completed through the first mapping catheter 102 and five first intracardiac waveforms are obtained, the frequency domain conversion is performed on each of the five first intracardiac waveforms obtained to obtain five first single-signal spectrograms. After the next collection is completed, the frequency domain conversion is performed on other five first intracardiac waveforms collected in the next collection to obtain five corresponding first single-signal spectrograms. By analogy, until all collections are completed, and the N first single-signal spectrograms can be obtained finally.

In an embodiment, before the frequency domain conversion is performed on each first intracardiac waveform, the method further includes: each first intracardiac waveform is pre-processed such that each first intracardiac waveform is within a preset frequency range. The preset frequency range can be set according to actual situations, for example, it can be a range of 0 to 20 Hz; and/or, after the frequency domain conversion is performed on each first intracardiac waveform, the method further includes: each first single-signal spectrogram is smoothed.

In other words, before the frequency domain conversion is performed on the first intracardiac waveform, the first intracardiac waveform can also be pre-processed. For example, the pre-processing step may include any one or any combination of QRS wave elimination, band-pass or high-pass filter processing, modulo operation, and low-pass filter processing. Then, the frequency domain conversion is performed on the pre-processed first intracardiac electrical signal to obtain the first single-signal spectrogram. In addition, after the first single-signal spectrogram is obtained, the obtained first single-signal spectrogram can also be smoothed. For example, the smoothing can be performed in manners of low-pass filter processing, complementary filtering, or Kalman filtering to obtain the smoother first single-signal spectrogram. The pre-processing and smoothing can be adopted alternatively or simultaneously.

As a specific example, as shown in FIG 4, the step of generating the first single-signal spectrogram of each first intracardiac waveform to obtain the N first single-signal spectrograms can further include steps S402, S404, and S406.

Step 402: each first intracardiac waveform is pre-processed to obtain N pre-processed first intracardiac waveforms in a preset frequency range, the pre-processing step may include any one or any combination of QRS wave elimination, band-pass or high-pass filter processing, modulo operation, and low-pass filter processing.

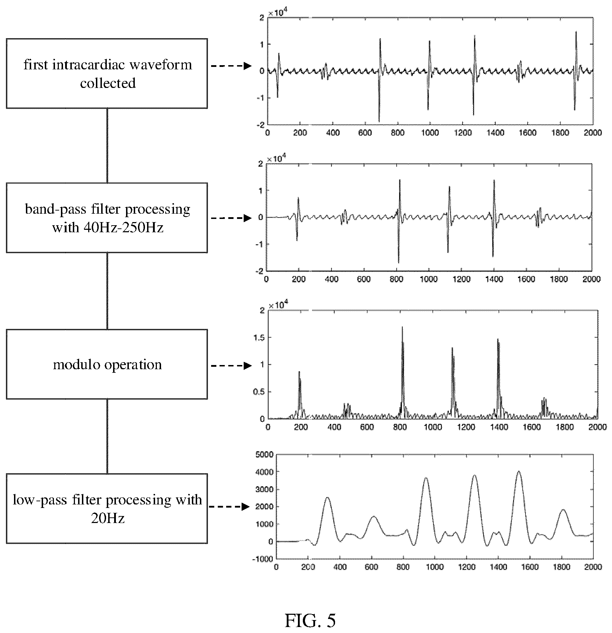

Specifically, after the first intracardiac waveform is obtained, the first intracardiac waveform can be pre-processed first. For example, referring to FIG 5, band-pass filter processing with a frequency of 40 Hz to 250 Hz can be firstly performed on the first intracardiac waveform. Then, a modulo operation can be performed on the first intracardiac waveform after the band-pass filter processing, such as an absolute value of negative intracardiac electrical signal is calculated, so that all intracardiac electrical signals are positive. Finally, the low-pass filter processing with a frequency of 0 Hz to 20 Hz is performed on the first intracardiac waveform after the modulo operation, so as to obtain the pre-processed first intracardiac waveform in the preset frequency range of 0 Hz to 20 Hz.

Step 404: the frequency domain conversion is performed on each pre-processed first intracardiac waveform to obtain N first original single-signal spectrograms; the Fast Fourier Transform (FFT) can be adopted to perform the frequency domain conversion on the first intracardiac waveform pre-processed. Here, a signal spectrogram after the frequency domain conversion represents the first original single-signal spectrogram.

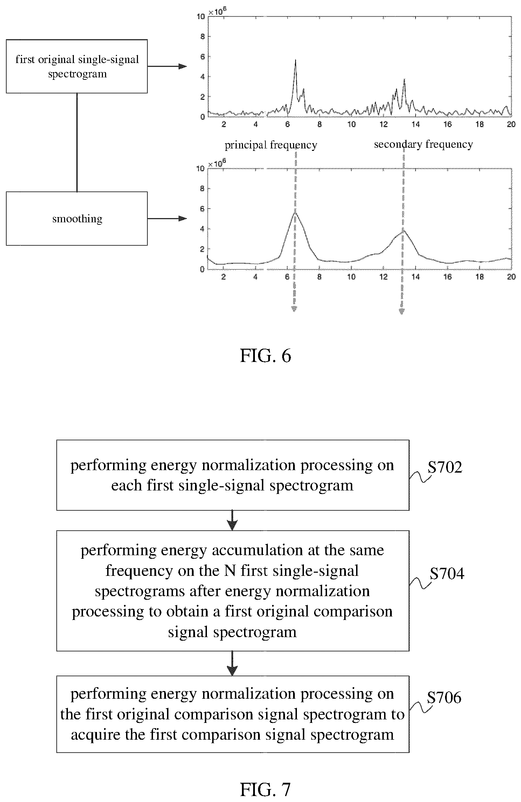

Step 406: each first original single-signal spectrogram is smoothed to obtain N first single-signal spectrograms. For example, referring to FIG 6, the first original single-signal spectrogram can be smoothed through the low-pass filter processing to obtain a smoother signal spectrogram, which is denoted as the first single-signal spectrogram.

Step 304: the N first single-signal spectrograms are combined to generate the first comparison signal spectrogram, or one of the N first single-signal spectrograms is selected as the first comparison signal spectrogram.

In other words, one first single-signal spectrogram can be directly acquired from the N first single-signal spectrograms as the first comparison signal spectrogram (in particular, when there is only one first single-signal spectrogram, the one first single-signal spectrogram serves as the first comparison signal spectrogram); or the N first single-signal spectrograms can also be combined, and a combined signal spectrogram serves as the first comparison signal spectrogram. In a preferred embodiment, the latter is adopted so that the intracardiac situation of the region of interest or the reference point/region can be reflected as a whole.

Specifically, when the N first single-signal spectrograms are combined to generate the first comparison signal spectrogram, the N first single-signal spectrograms are combined after the collection of the N first single-signal spectrograms is completed, to generate the first comparison signal spectrogram; or after each collection is completed and the corresponding first single-signal spectrogram is obtained, the obtained first single-signal spectrograms are combined. In a preferred embodiment, the latter is adopted, so that the problem of long analysis time caused by the centralized data processing can be effectively reduced, and the real-time performance of the signal spectrogram can be ensured.

For example, when the number of electrodes of the first mapping catheter 102 is five, after the first collection through the first mapping catheter 102 is completed and five first intracardiac waveforms are obtained, the frequency domain conversion is performed on each of the five obtained first intracardiac waveforms, to obtain five first single-signal spectrograms; and the five obtained first single-signal spectrograms are combined to obtain one signal spectrogram. Then, after the next collection is completed, the frequency domain conversion is performed on other five first intracardiac waveforms collected in the next collection to obtain five first single-signal spectrograms. Then the five first single-signal spectrograms are combined to the signal spectrogram previously obtained to obtain one new signal spectrogram. By analogy, until the combination of the N first single-signal spectrograms is completed, the first comparison signal spectrogram is obtained finally.

In an embodiment, the step of combining the N first single-signal spectrograms to generate the first comparison signal spectrogram further includes: accumulation processing is performed on the N first single-signal spectrograms; the accumulation processing can further include: a curve accumulation method or a probabilistic method. That is, the curve accumulation method or probabilistic method can be adopted to perform the accumulation processing on the N first single-signal spectrograms to generate the first comparison signal spectrogram.

In an embodiment, as shown in FIG 7, the step of performing the accumulation processing on the N first single-signal spectrograms to generate the first comparison signal spectrogram by the curve accumulation method can further include steps S702, S704, and S706.

Step 702: energy normalization processing is performed on each first single-signal spectrogram.

**In** an embodiment, when the energy normalization processing is performed on each first single-signal spectrogram, features such as a frequency and an energy corresponding to the frequency can be extracted first for each first single-signal spectrogram, and the frequencies are sorted in order according to the energy corresponding to the frequency from most to least. The frequency corresponding to the most energy is defined as a first frequency (dominant frequency), and the corresponding energy is defined as a first frequency energy (dominant frequency energy). At the same time, the frequency corresponding to the least energy is removed, and then the energy normalization processing is performed.

For example, after the N first single-signal spectrograms are obtained, the features of each first single-signal spectrogram can be extracted, such as the first frequency (dominant frequency), the first frequency energy (dominant frequency energy), a second frequency (secondary frequency), a second frequency energy (secondary frequency energy), a third frequency, a third frequency energy,..., an *i-th* frequency, an *i-th* frequency energy. Here, *i* is taken to sequentially mark according to the magnitude of energy. The frequency at the maximum energy is the first frequency, i.e., the dominant frequency; the frequency at the sub-maximum energy is the second frequency, i.e., a secondary frequency, and so on. The value of i is comprehensively considered according to the complexity of the disease and related energy. Generally, the more complex the disease, the greater the value of *i*. At the same time, the energy of some frequencies is too low, such as less than 10% of the first frequency energy, it can be ignored. Then, the energy normalization processing can be performed on the first frequency energy, the second frequency energy, the third frequency energy, ..., the *i-th* frequency energy of each first single-signal spectrogram, that is, the energy is limited to 1 or less.

Step 704: a same-frequency energy accumulation is performed on the N first single-signal spectrograms after the energy normalization processing to obtain a first original comparison signal spectrogram.

The same-frequency energy accumulation refers to an energy accumulation corresponding to the same frequency between different first single-signal spectrograms. For example, in the N first single-signal spectrograms, there are two signal spectrograms with the first frequency of 5 Hz, then after the same-frequency energy accumulation is performed, the energy corresponding to frequency of 5 Hz is a sum of the energies of the two signal spectrograms. As another example, in the N first single-signal spectrograms, there are three signal spectrograms with the first frequency of 5 Hz, then after the same-frequency energy accumulation, the energy corresponding to frequency of 5 Hz is a sum of the energies corresponding to the first frequencies of the three signals. In such a way, the energy accumulations of all frequencies are completed, and a new signal spectrogram can finally be formed, which is denoted as the first original comparison signal spectrogram.

Step 706: the energy normalization processing is performed on the first original comparison signal spectrogram to obtain the first comparison signal spectrogram.

Specifically, after the first original comparison signal spectrogram is obtained, the energy normalization processing is further performed on the first original comparison signal spectrogram. The specific process is the same as the process of performing the energy normalization processing on each first single-signal spectrogram. For example, the features of the first original comparison signal spectrogram can be extracted first, such as the first frequency (dominant frequency), the first frequency energy (dominant frequency energy), the second frequency (secondary frequency), the second frequency energy (secondary frequency energy), the third frequency, the third frequency energy,..., a *j-th* frequency, a *j-th* frequency energy. Here *j* is taken to sequentially mark according to the magnitude of the energy. The frequency at the maximum energy is the first frequency which is the dominant frequency, the frequency at the sub-maximum energy is the second frequency which is the secondary frequency, and so on. Then, the energy normalization processing is performed on the first frequency energy, the second frequency energy, the third frequency energy, ..., the *j-th* frequency energy which are extracted, to obtain the first comparison signal spectrogram.

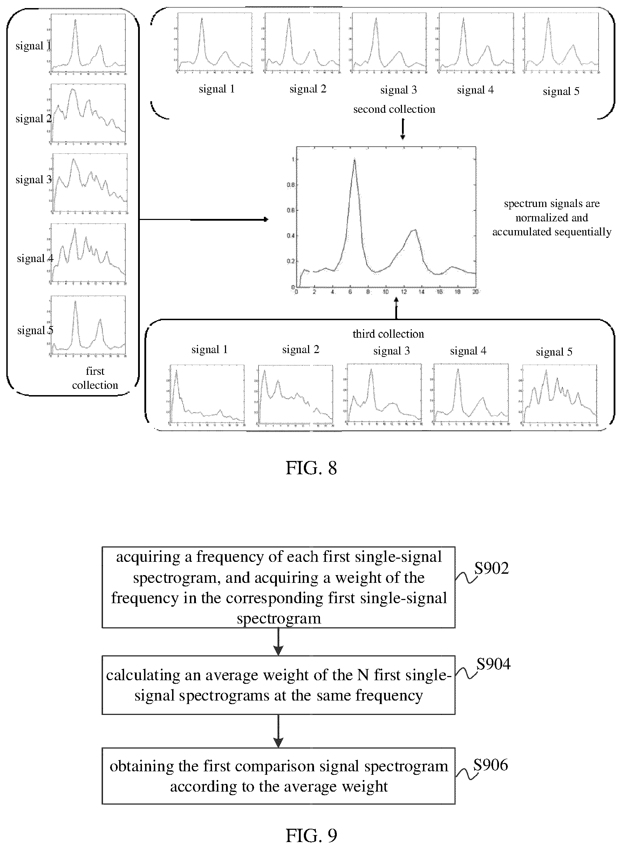

As a specific example, when the number of electrodes of the first mapping catheter 102 is five, after one collection through the first mapping catheter 102 is completed and five first intracardiac waveforms are obtained, the frequency domain conversion is performed on each of the five obtained first intracardiac waveforms to obtain five first single-signal spectrograms; and the energy normalization processing is performed on each of the five obtained first single-signal spectrograms; the same-frequency energy accumulation and energy normalization processing are performed on the five normalized first single-signal spectrograms, to obtain one signal spectrogram. Then, after the next collection is completed, other five first single-signal spectrograms are obtained. Similarly, the energy normalization processing is performed on the five first single-signal spectrograms; and the same-frequency energy accumulation and energy normalization processing are performed with the previously obtained signal spectrogram to obtain one new signal spectrogram. By analogy, until the same-frequency energy accumulation and energy normalization processing for the N first single-signal spectrograms are completed, the first comparison signal spectrogram is obtained finally, as shown in FIG 8. At this point, the acquisition of the first comparison signal spectrogram of the region of interest or the reference point/region is completed.

In another embodiment, as shown in FIG 9, the step of performing the accumulation processing on the N first single-signal spectrograms by the probabilistic method to generate the first comparison signal spectrogram can further include steps S902, S904, and S906.

Step 902: a frequency of each first single-signal spectrogram is acquired, and a weight of the frequency in a corresponding first single-signal spectrogram is acquired.

The acquisition process of the frequency is the same as the acquisition process in the embodiments corresponding to FIG 7 and FIG 8, and the details are not repeated here. After the frequency of each first single-signal spectrogram (such as the first frequency (dominant frequency), the second frequency (secondary frequency), the third frequency, ..., a *i-th* frequency) is acquired through the aforementioned manner, a weight of each frequency in a corresponding first single-signal spectrogram is obtained. The weight can be determined according to a frequency area, a harmonic frequency area of the frequency, and an entire spectrogram area. For example, the weight of the first frequency equals to a sum of the frequency area of the first frequency and the harmonic frequency area of the first frequency divided by the entire spectrogram area. As shown in Table 1, in the first spectrogram of the first single-signal spectrograms, after calculation, the weight of the first frequency in the first single-signal spectrogram is equal to 0.33.

Step 904: an average weight at the same frequency in the N first single-signal spectrograms is calculated.

The average weight at the same frequency refers to an average weight value corresponding to the same frequency order (for example, all the same frequencies are the first frequencies) between different first single-signal spectrograms. Referring to Table 1, for the first frequency, the average weight = (0.33 + 0.35 + 0.36 + 0.31 + 0.32 + 0.32 + 0.36 + 0.36 + 0.36 + 0.31 + 0.31 + 0.24) /15=0.28. In the first frequency, the weight corresponding to frequency of 1.8 is ignored, that is, when the average weight is calculated, if the value of frequency of i is the same (for example, all are the first frequencies), but the actual acquired frequency values (such as 6.4 and 1.8) are different, the weight corresponding to the actually obtained smaller frequency value (in this embodiment, it is 1.8) is replaced with zero.

Step 906: the first comparison signal spectrogram is obtained according to the average weight.

Specifically, as shown in Table 1, after the average weight is obtained, the obtained first frequency of the first comparison signal spectrogram is equal to 6.40, the weight corresponding to the first frequency is equal to 0.28, the second frequency is equal to13.20, the weight corresponding to the second frequency is equal to 0.10, ..., thus the first comparison signal spectrogram can be obtained.

**Table 1**

| Collection number | Collection point | The first frequency | | The second frequency | | The third frequency | | The fourth frequency | | The fifth frequency | |
|---|---|---|---|---|---|---|---|---|---|---|---|
| | | frequency (HZ) | weight | frequency (HZ) | weight | frequency (HZ) | weight | frequency (HZ) | weight | frequency (HZ) | weight |
| First collection | 1. | 6.40 | 0.33 | 9.20 | 0.18 | 3.00 | 0.18 | 10.40 | 0.17 | 15.20 | 0.15 |
| | 2. | 6.40 | 0.35 | 10.40 | 0.18 | 2.10 | 0.17 | 16.50 | 0.10 | / | / |
| | 3. | 6.40 | 0.36 | 10.40 | 0.17 | 2.10 | 0.17 | 16.50 | 0.10 | / | / |
| | 4. | 6.40 | 0.31 | 13.20 | 0.26 | / | / | / | / | / | / |
| | 5. | 6.40 | 0.32 | 13.20 | 0.18 | / | / | / | / | / | / |
| Second collection | 1. | 6.40 | 0.32 | 13.20 | 0.18 | / | / | / | / | / | / |
| | 2. | 6.40 | 0.36 | 13.20 | 0.14 | / | / | / | / | / | / |
| | 3. | 6.40 | 0.36 | 13.20 | 0.14 | 3.00 | 0.10 | / | / | / | / |
| | 4. | 6.40 | 0.36 | 13.20 | 0.14 | 3.00 | 0.10 | / | / | / | / |
| | 5. | 6.40 | 0.31 | 13.20 | 0.20 | / | / | / | / | / | / |
| Third collection | 1. | 6.40 | 0.31 | 13.20 | 0.20 | / | / | / | / | / | / |
| | 2. | 6.40 | 0.31 | 1.80 | 0.14 | 13.20 | 0.10 | / | / | / | / |
| | 3. | 1.80 | 0.28 | 5.20 | 0.22 | / | / | / | / | / | / |
| | 4. | 1.80 | 0.44 | 13.20 | 0.11 | / | / | / | / | / | / |
| | 5. | 6.40 | 0.24 | 9.20 | 0.22 | 3.00 | 0.18 | 15.20 | 0.14 | / | / |
| Accumulat -ion result | | 6.40 | 0.28 | 13.20 | 0.10 | 3.00 | 0.04 | | | | |

As a specific example, referring to Table 1, when the number of electrodes of the first mapping catheter 102 is five, after the first collection through the first mapping catheter 102 is completed and five first intracardiac waveforms are obtained, the frequency domain conversion is performed on each of the five obtained first intracardiac waveforms to obtain five first single-signal spectrograms, and the features of each of the five obtained first single-signal spectrograms are extracted, such as extracting the first frequency, the second frequency, the third frequency, the fourth frequency and the fifth frequency; and the weight of each frequency in the corresponding first single-signal spectrogram is calculated. For example, at the collection point 1, the weight corresponding to the first frequency of 6.40 Hz is equal to 0.33, and the weight corresponding to the second frequency of 9.2 Hz is equal to 0.18. Then, the weights corresponding to the five first frequencies of 6.4 Hz are added and averaged. Then, after the next collection is completed, other five first single-signal spectrograms are obtained. After the same processing, i.e., accumulating and averaging operations are performed, and so on, and finally the first comparison signal spectrogram with an energy distribution from large to small is obtained. At this point, the acquisition of the first comparison signal spectrogram of the region of interest or the reference point/region is completed.

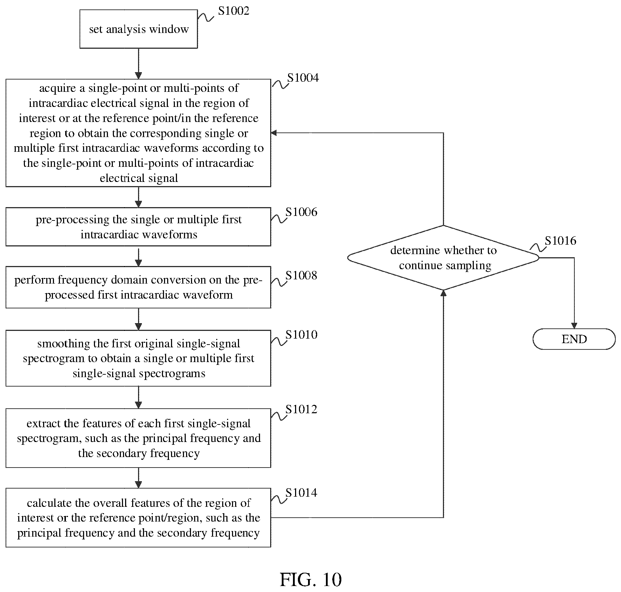

FIG 10 is a flow chart showing acquisition of a first comparison signal spectrogram in an embodiment, as shown in FIG 10, the process of acquiring the first comparison signal spectrogram can include the following steps.

Step S1002: collection time is set through an analysis window; for example, the collection time is set to 10s through the analysis window.

Step S1004: a single-point or multi-point intracardiac electrical signal in the region of interest or at the reference point/in the reference region is acquired to obtain a corresponding single or multiple first intracardiac waveforms according to the single-point or multi-point intracardiac electrical signal.

Step S1006: the single or multiple first intracardiac waveforms are pre-processed, such as any one or any combination of QRS wave elimination, band-pass or high-pass filter processing, a modulo operation, and low-pass filter processing.

Step S1008: the frequency domain conversion is performed on the pre-processed first intracardiac waveform, such as performing FFT, to obtain the first original single-signal spectrogram.

Step S 1010: the first original single-signal spectrogram is smoothed to obtain a single or multiple first single-signal spectrograms, such as using a low-pass filtering method to smooth.

Step S1012: a feature of each first single-signal spectrogram is extracted, such as the dominant frequency or the secondary frequency.

Step S1014: a global feature of the region of interest or the reference point/region is calculated, such as the dominant frequency or the secondary frequency.

Step S1016: whether to continue the collection is determined. If the collection is continued, return to the step S1004; if the collection is not continued, the acquisition process ends, and the acquisition of the first comparison signal spectrogram of the region of interest or the reference point/region is completed.

In an embodiment, the first comparison signal spectrogram and the second comparison signal spectrogram are generated in the same manner.

Specifically, the step of generating the second comparison signal spectrogram according to the M second intracardiac waveforms can further include: a second single-signal spectrogram of each second intracardiac waveform is generated to obtain M second single-signal spectrograms; and the M second single-signal spectrograms are combined to generate the second comparison signal spectrogram, or one second single-signal spectrogram is selected from the M second single-signal spectrograms as the second comparison signal spectrogram. The step of generating the second single-signal spectrogram of each second intracardiac waveform is the same as the step of generating the first single-signal spectrogram of each first intracardiac waveform. The step of combining the M second single-signal spectrograms to generate the second comparison signal spectrogram is the same as the step of combining the N first single-signal spectrograms to generate the first comparison signal spectrogram. In particular, when there is only one second single-signal spectrogram, the second single-signal spectrogram serves as the second comparison signal spectrogram.

That is, for the process of acquiring the second comparison signal spectrogram of the point of interest, reference can be made to the process of acquiring the first comparison signal spectrogram of the region of interest or the reference point/region. For example, in the case where there are a plurality of second intracardiac waveforms, for the acquisition of the second comparison signal spectrogram of the point of interest, reference can be made to steps S1002 to S1016, and details will not be repeated here.

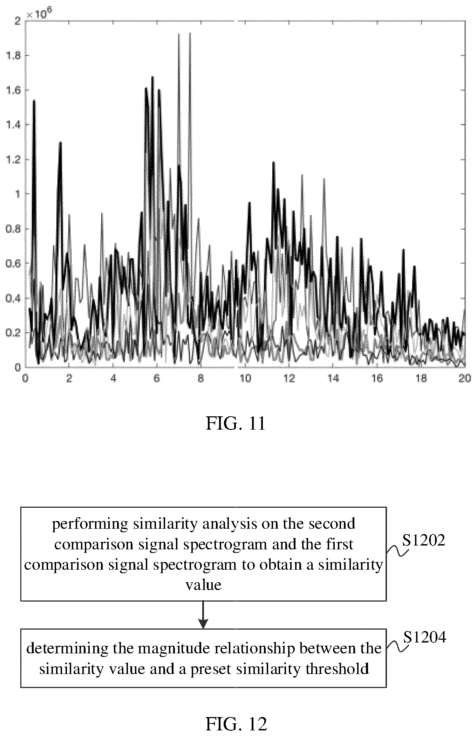

In an embodiment, the step of combining the N first single-signal spectrograms to generate the first comparison signal spectrogram further includes: the N first single-signal spectrograms are superimposed. The superimposing refers to simply superimposing the N first single-signal spectrograms. For example, FIG 11 shows a signal spectrogram generated by superimposing five first single-signal spectrograms, which represents the first comparison signal spectrogram.

In the above embodiment, the manner of acquiring the second comparison signal spectrogram of the point of interest can be selected according to actual needs: one single-signal spectrogram can be directly selected from the acquired single-signal spectrograms as the comparison signal spectrogram; the specific selection mode can be made by the doctor, for example, sending a selection instruction to a computer system to select one single-signal spectrogram; the single-signal spectrograms can also be combined through accumulation or superposition mode to obtain a comparison signal spectrogram.

In an embodiment, as shown in FIG 12, the step of performing comparative analysis on the first comparison signal spectrogram and the second comparison signal spectrogram can further include steps S1202 and S1204.

Step 1202: a similarity analysis is performed on the second comparison signal spectrogram and the first comparison signal spectrogram to obtain a similarity value. For example, when the similarity analysis is performed, the Pearson correlation coefficient can be adopted to represent the similarity value, or other methods can also be adopted to perform the similarity analysis.

Step 1204: a magnitude relationship between the similarity value and a preset similarity threshold is determined. The preset similarity threshold can be set according to actual conditions, for example, the similarity threshold can be 0.6.

Specifically, whether the point of interest is the focal point can be determined by comparing the curve similarity of the first comparison signal spectrogram and the second comparison signal spectrogram. For example, a curve similarity analysis can be performed on the first comparison signal spectrogram and the second comparison signal spectrogram; and the similarity analysis can be represented by a Pearson correlation coefficient. If the analyzed similarity value exceeds the preset similarity threshold such as 0.6, it can be determined that the point of interest is the focal point. In this case, prompt information indicating that the point of interest is the focal point can be output in the manner of a text, an image, or voice. For example, the similarity value and the preliminary determination result can be displayed in a text, and can also be played in voice, and the portion with a higher similarity in the second comparison signal spectrogram can be marked to remind the doctor. The specific manner can be selected according to actual needs. If the analyzed similarity value does not exceed the preset similarity threshold such as 0.6, it can be determined that the point of interest is a non-focal point. In this case, the prompt information indicating that the point of interest is a non-focal point can be output in the manner of a text, an image, or voice.

In an embodiment, there is a plurality of the points of interest, thereby generating a plurality of the second comparison signal spectrograms. The step of performing the comparative analysis on the first comparison signal spectrogram and the second comparison signal spectrogram and generating the comparative analysis result can further include: the comparative analysis is performed on the first comparison signal spectrogram and the plurality of the second comparison signal spectrograms, respectively, thereby generating the comparative analysis result.

It can be understood that, after a second comparison signal spectrogram of one point of interest is obtained in the above manner, second comparison signal spectrograms of other points of interest can be continuously obtained, and then one second comparison signal spectrogram with the highest similarity to the first comparison signal spectrogram is acquired from the plurality of second comparison signal spectrograms obtained, and the point of interest corresponding to the second comparison signal spectrogram is taken as the focal point.

In this embodiment, by analyzing the corresponding relationship between the signal spectrogram of the region of interest or the reference point/region and the signal spectrogram of the point of interest, that is, by analyzing the corresponding relationship between the global and local spectrograms, the relevant rules are found out, thereby providing the determination result of the focal point as a whole to provide a medical advice to the doctor in order to help the doctor to obtain more valuable intracardiac information, which is beneficial to improve the accuracy of ablation, thereby improving the cure rate of arrhythmia diseases, and meanwhile avoiding unnecessary injury of the patient.

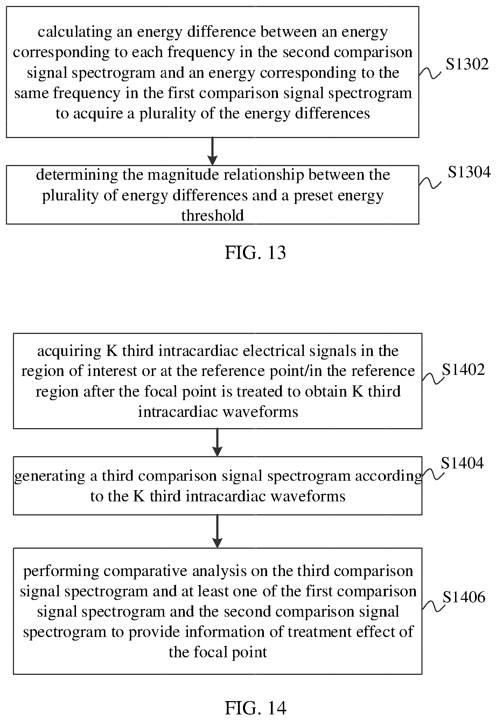

In an embodiment, as shown in FIG 13, the step of performing the comparative analysis on the first comparison signal spectrogram and the second comparison signal spectrogram can further include steps S1302 and S 1304.

Step 1302: an energy difference between an energy corresponding to each frequency in the second comparison signal spectrogram and an energy corresponding to the same frequency in the first comparison signal spectrogram, to obtain a plurality of energy differences.

Step 1304: a magnitude relationship between the plurality of energy differences and a preset energy threshold; the preset energy threshold can be selected according to the actual situation, for example, the preset energy threshold can be a normalized energy corresponding to the first frequency of the region of interest.

Specifically, whether the point of interest is a focal point can be determined by comparing the energies corresponding to the frequencies of the first comparison signal spectrogram and the second comparison signal spectrogram. For example, when the difference value between the energy corresponding to a certain frequency (such as a dominant frequency) in the second comparison signal spectrogram and the energy corresponding to the same frequency (such as the dominant frequency) in the first comparison signal spectrogram exceeds the preset energy threshold, it can be determined that the point of interest is the focal point. At this time, the prompt information indicating that the point of interest is the focal point can be output in the manner of a text, an image, or voice. For example, the frequency energy of the point of interest and the preliminary determination result can be displayed in a text, or can also be played in voice, or a portion in the second comparison signal spectrogram with a higher energy can be marked to remind the doctor. The specific manner can be selected according to actual needs. Otherwise, when the difference value between the energy corresponding to a certain frequency (such as the dominant frequency) in the second comparison signal spectrogram and the energy corresponding to the same frequency (such as the dominant frequency) in the first comparison signal spectrogram does not exceed the preset energy threshold, it can be determined that the point of interest is a non-focal point. In this case, similarly, the prompt information indicating that the point of interest is a non-focal point can be output in the manner of a text, an image, or voice.

It should be noted that when it is determined whether the point of interest is a focal point, the above two manners can also be combined, that is, both the certain frequency energy and the curve similarity of the spectrograms are considered. For example, the two can be comprehensively analyzed by assigning a certain weight, which will not be detailed here.

In this embodiment, by analyzing the corresponding relationship between the signal spectrogram of the region of interest or the reference point/region and the signal spectrogram of the point of interest, that is, by analyzing the corresponding relationship between the global and local spectrograms, the relevant rules are found out, thereby providing the determination result of the focal point as a whole to provide a medical advice to the doctor to help the doctor to obtain more valuable intracardiac information, which is beneficial to improve the cure rate of the arrhythmia diseases.

Further, after the determination of whether the point of interest is the focal point is completed, the determination result can be presented to the doctor. In such a way, the doctor can make further comparison and determination based on the determination result and combined with the first comparison signal spectrogram and the second comparison signal spectrogram, to determine whether to treat the point of interest as a focal point. The treatment means of arrhythmia diseases is usually to physically destroy the heart muscle. For example, the myocardium can be inactivated by radio frequency ablation energy, or the myocardium can be inactivated by freezing, laser, ultrasound, etc., to achieve the purpose of treating the arrhythmia disease.

In an embodiment, as shown in FIG 14, the method for analyzing an ECG signal can further include steps S1402, S1404, and S1406.

Step 1402: K third intracardiac electrical signals in the region of interest or at the reference point/in the reference region after the focal point is treated are acquired, and then K third intracardiac waveforms are obtained, K is an integer greater than or equal to 1.

Step 1404: a third comparison signal spectrogram is generated according to the K third intracardiac waveforms (a signal spectrogram corresponding to the region of interest or the reference point/region after the treatment represents the third comparison signal spectrogram).

In an embodiment, the third comparison signal spectrogram and the first comparison signal spectrogram are generated in the same manner.

Specifically, the step of generating the third comparison signal spectrogram according to the K third intracardiac waveforms can further include: a third single-signal spectrogram of each third intracardiac waveform is generated to obtain the K third single-signal spectrograms, and the K third single-signal spectrograms are combined to generate the third comparison signal spectrogram, or one third single-signal spectrogram is selected from the K third single-signal spectrograms as the third comparison signal spectrogram. The step of generating the third single-signal spectrogram of each third intracardiac waveform is the same as the step of generating the first single-signal spectrogram of each first intracardiac waveform. The step of combining the K third single-signal spectrograms to generate the third comparison signal spectrogram is the same as the step of combining the N first single-signal spectrograms to generate the first comparison signal spectrogram. In particular, when there is only one third single-signal spectrogram, the third single-signal spectrogram serves as the third comparison signal spectrogram.

For the specific generating process, reference can be made to the process of generating the first comparison signal spectrogram, which will not be repeated here.

Step 1406: the comparative analysis is performed on the third comparison signal spectrogram and at least one of the first comparison signal spectrogram and the second comparison signal spectrogram to provide information of a treatment effect of the focal point.

Specifically, after the treatment of the focal point is completed, the treatment effect can be determined by comparing the comparison signal spectrograms of the region of interest or the reference point/region before and after the treatment. Since the position of the reference point/region does not change during the entire treatment process, after the completion of one treatment, it is preferable to compare and determine the comparison signal spectrograms of the reference point/region before and after the treatment to determine whether the treatment is effective, and whether to perform further processing according to whether the treatment is effective. After the above treatment, the following situations may appear: 1) a certain frequency energy (such as the dominant frequency energy) disappears on the third comparison signal spectrogram of the reference point/region, and the arrhythmia restores the sinus rhythm; 2) a certain frequency energy (such as the dominant frequency energy) is weakened on the third comparison signal spectrogram of the reference point/region, and the sinus rhythm is not restored, there may be other points of interest with frequency energies, the mapping catheter is continuously utilized to find; or 3) a certain frequency energy (such as the dominant frequency energy) does not change on the third comparison signal spectrogram of the reference point/region, and the sinus rhythm is not restored, the mapping catheter is continuously utilized to find, or the treatment has no other isolation effects, the ablation is continuously optimized. The above process is continuously cycled until the treatment of arrhythmia disease is completed.

Complex arrhythmia diseases may usually have multiple driving frequencies, so the principal contradiction, that is, the dominant frequency, can be resolved first. If the disease still recurs after the resolution of the dominant frequency, the secondary frequency becomes the principal contradiction; and at this time the secondary frequency rises to be the dominant frequency, and thus enters the next cycle, and so on, until the treatment of arrhythmia disease is completed and the sinus rhythm is restored.

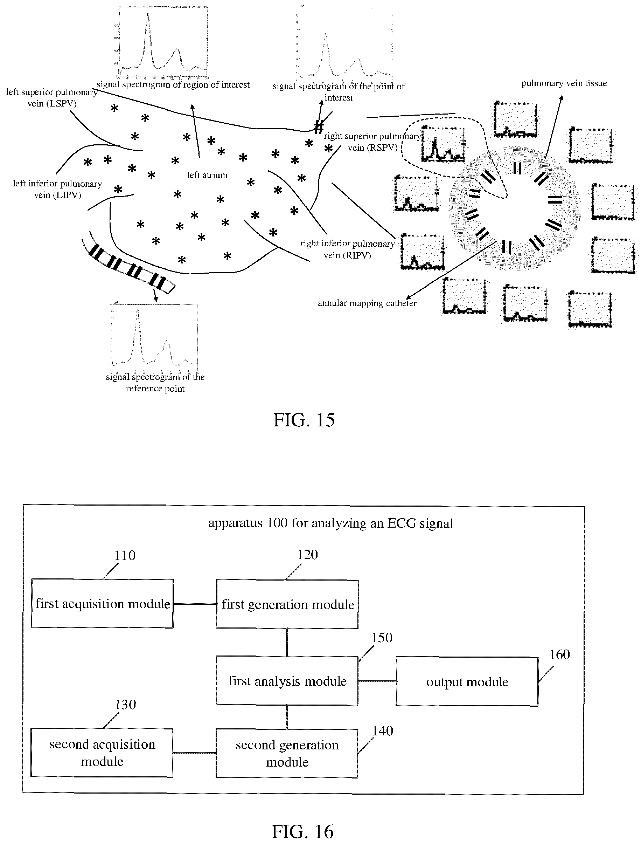

The treatment of atrial fibrillation is taken as an example, the region of interest for the atrial fibrillation is usually in the left atrium. As shown in FIG 15, the left atrium can be mapped and the comparison signal spectrogram of the region of interest can be obtained. In an atrial fibrillation operation, because the mapping catheter placed at the coronary sinus remains immobile throughout the operation, an intracardiac electrical signal can be used as the reference point and the comparison signal spectrogram at the reference point can be obtained. There is a high probability that the focal point of the atrial fibrillation comes from four pulmonary veins, therefore these regions can be preferentially mapped to obtain the comparison signal spectrogram of the point of interest.

In this embodiment, as shown in FIG 15, an annular mapping catheter with twenty electrodes can be adopted. This mapping catheter can simultaneously collect ten sets of intracardiac electrical signals through the twenty electrodes, which can be divided into ten points in the region of pulmonary vein, and spectrogram analysis can be performed respectively to obtain comparison signal spectrograms of ten points of interest. Then, the comparison signal spectrograms of the ten points of interest are respectively compared to the comparison signal spectrogram of the region of interest and the determination is performed, such as the curve similarity comparison or frequency energy comparison, to determine whether the point of interest is the focal point. Referring to FIG 15, the comparison signal spectrogram of the point of interest within the dotted line has the highest similarity to the comparison signal spectrogram of the region of interest, so this point can be determined as the focal point. In this case, the doctor can treat the point according to the determination result; and after the treatment is completed, the comparison signal spectrogram of the reference point is obtained again and is compared to the comparison signal spectrogram of the reference point before the treatment with the determination, such as the curve similarity comparison or frequency energy comparison, to determine the treatment effect.

After one treatment is completed, if the treatment effect is not favorable, then the point of interest is changed and the treatment is continued, until the frequency energy on the comparison signal spectrogram of the region of interest or the reference point/region after treatment disappears, relative to the frequency energy on the comparison signal spectrogram of the region of interest or the reference point/region before the treatment, and then the arrhythmia restores the sinus rhythm.

In this embodiment, by comparing and analyzing the relationship between the global and local spectrograms, the intracardiac spectrogram can be comprehend as a whole, so that the location of the focal point can be found more accurately, and the damage to healthy heart muscle tissue can be effectively reduced while improving the cure rate of the arrhythmia diseases.

It should be understood that although the steps in the flowcharts of FIGS. 2 to 4, 7, 9 to 10, and 12 to 14 are displayed in order according to the arrows, the steps are not definitely executed in the order indicated by the arrows. Unless clearly stated in this article, the execution of these steps is not strictly limited in order, and these steps can be executed in other orders. Moreover, at least some of the steps in FIGS. 2 to 4, 7, 9 to 10, and 12 to 14 may include multiple sub-steps or multiple stages. These sub-steps or stages are not definitely executed at the same time, but may be performed at different time, the execution order of these sub-steps or stages is not definitely sequential, but may be executed in turns or alternately with at least a part of other steps or sub-steps or stages of other steps.

In an embodiment, as shown in FIG 16, an apparatus 100 for analyzing an ECG signal is provided, including: a first acquisition module 110, a first generation module 120, a second acquisition module 130, a second generation module 140, a first analysis module 150, and an output module 160.

The first acquisition module 110 can be connected to an external signal collection device (such as a electrophysiological mapping catheter), and is configured to acquire N first intracardiac electrical signals in a region of interest or at a reference point/in a reference region to obtain N first intracardiac waveforms; N is an integer greater than or equal to 1.

The first generation module 120 is configured to generate a first comparison signal spectrogram according to the N first intracardiac waveforms.

The second acquisition module 130 can be connected to an external signal collection device (such as an electrophysiological mapping catheter), and is configured to acquire M second intracardiac electrical signals at a point of interest to acquire M second intracardiac waveforms; M is an integer greater than or equal to 1.

The second generation module 140 is configured to generate a second comparison signal spectrogram according to the M second intracardiac waveforms.

The first analysis module 150 is configured to perform a comparative analysis on the first comparison signal spectrogram and the second comparison signal spectrogram, and generate a comparative analysis result.

The output module 160 is configured to output prompt information indicating whether the point of interest is a focal point according to the comparative analysis result.

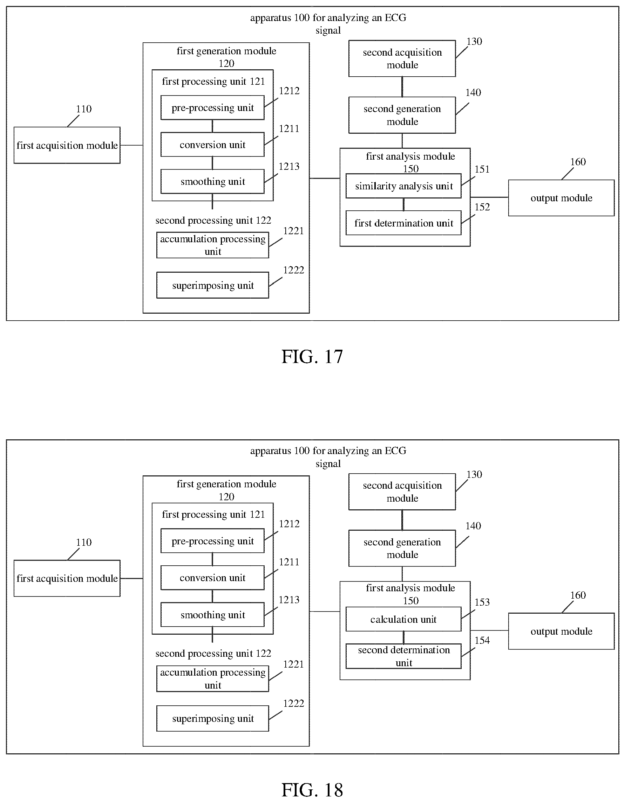

In an embodiment, as shown in FIG 17, the first generation module 120 further includes:
a first processing unit 121, configured to generate a first single-signal spectrogram of each first intracardiac waveform to obtain N first single-signal spectrograms;
a second processing unit 122, configured to combine the N first single-signal spectrograms to generate a first comparison signal spectrogram, or select one first single-signal spectrogram from the N first single-signal spectrograms as the first comparison signal spectrogram.

In an embodiment, as shown in FIG 17, the first processing unit 121 further includes:
a conversion unit 1211, configured to perform a frequency domain conversion on each first intracardiac waveform to acquire the N first single-signal spectrograms.

In an embodiment, as shown in FIG 17, the first processing unit 121 further includes:
a pre-processing unit 1212, configured to pre-process each first intracardiac waveform before performing the frequency domain conversion on each first intracardiac waveform, to make each first intracardiac waveform within a preset frequency range;
a smoothing unit 1213, configured to smooth each first single-signal spectrogram after performing the frequency domain conversion on each first intracardiac waveform.

In an embodiment, as shown in FIG 17, the second processing unit 122 further includes:
an accumulation processing unit 1221, configured to perform accumulation processing on the N first single-signal spectrograms to generate the first comparison signal spectrogram; here the accumulation processing includes a curve accumulation method or a probabilistic method.

In an embodiment, as shown in FIG 17, the second processing unit 122 further includes:
a superimposing unit 1222, configured to superimpose the N first single-signal spectrograms to generate the first comparison signal spectrogram.

In an embodiment, as shown in FIG 17, the first analysis module 150 further includes:
a similarity analysis unit 151, configured to perform a similarity analysis on the second comparison signal spectrogram and the first comparison signal spectrogram to obtain a similarity value;
a first determination unit 152, configured to determine a magnitude relationship between the similarity value and a preset similarity threshold.

In an embodiment, as shown in FIG 18, the first analysis module 150 further includes:
a calculation unit 153, configured to calculate an energy difference between an energy corresponding to each frequency in the second comparison signal spectrogram and an energy corresponding to a same frequency in the first comparison signal spectrogram to obtain a plurality of energy differences;
a second determination unit 154, configured to determine a magnitude relationship between the plurality of energy differences and a preset energy threshold.

In an embodiment, the output module 160 outputs prompt information indicating whether the point of interest is the focal point in the manner of a text, an image, or voice.

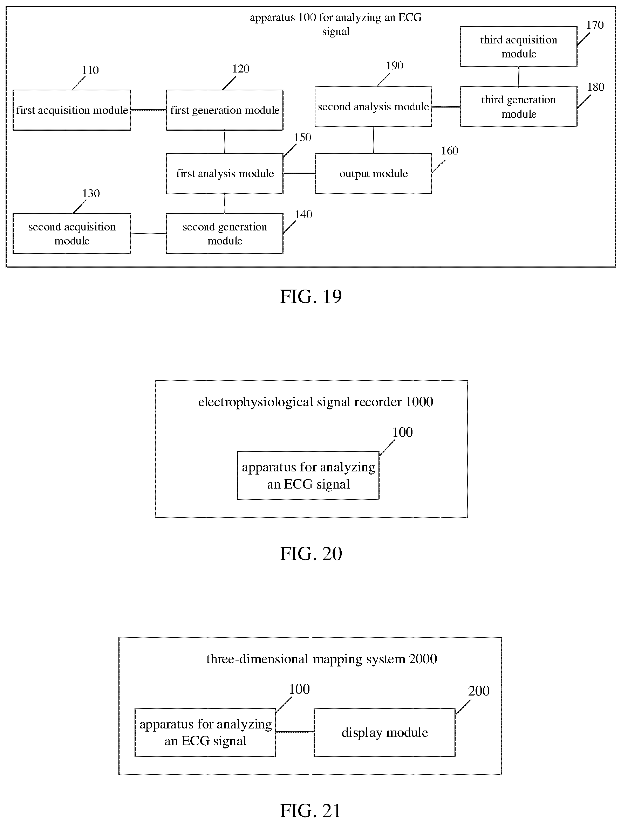

In an embodiment, as shown in FIG 19, the apparatus 100 for analyzing an ECG signal further includes:
a third acquisition module 170, configured to acquire K third intracardiac electrical signals in the region of interest or at the reference point/in the reference region after the focal point is treated, to obtain K third intracardiac waveforms, here K is an integer greater than or equal to 1;
a third generation module 180, configured to generate a third comparison signal spectrogram according to the K third intracardiac waveforms;
a second analysis module 190, configured to perform a comparative analysis on the third comparison signal spectrogram and at least one of the first comparison signal spectrogram and the second comparison signal spectrogram to provide information of a treatment effect of the focal point.

In an embodiment, the second generation module 140 and/or the third generation module 180 and the first generation module 120 are a same function module; the second acquisition module 130 and/or the third acquisition module 170 and the first acquisition module 110 are a same function module; the first analysis module 150 and the second analysis module 190 are a same function module.

For specific limit of the apparatus 100 for analyzing an ECG signal, reference can be made to the above limit of the method for analyzing an ECG signal, and details are not repeated herein again. Each module in the above-mentioned apparatus 100 for analyzing an ECG signal may be implemented in whole or in part by software, hardware, or a combination thereof. The above modules may be embedded in the hardware or independent of the processor in a computer device, or may be stored in a memory in the computer device in the form of software, so that the processor can call and execute the operations corresponding to the above modules.

In an embodiment, an electrophysiological signal recorder 1000 is provided. As shown in FIG 20, the electrophysiological signal recorder 1000 includes the aforementioned apparatus 100 for analyzing an ECG signal.

In an embodiment, a three-dimensional mapping system 2000 is provided, as shown in FIG 21, the three-dimensional mapping system 2000 includes the aforementioned apparatus 100 for analyzing an ECG signal, and a display module 200 configured to display a three-dimensional model of a heart; the output module 160 is configured to output the prompt information to the display module 200, and the prompt information is displayed on the three-dimensional model of the heart.

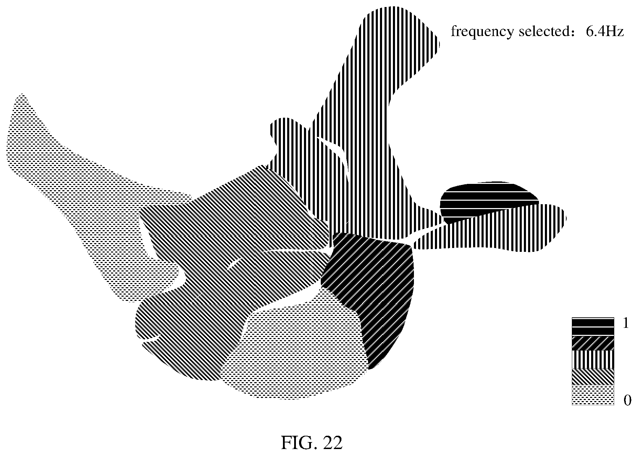

In an embodiment, the apparatus 100 for analyzing an ECG signal can be embedded in the three-dimensional mapping system 2000 to provide the doctor with more favorable information in the manner of regionalized display of the three-dimensional graphics of the heart. Referring to FIG 22, the apparatus 100 for analyzing an ECG signal acquires and saves the first single-signal spectrogram of all collection points in the region of interest, and extracts the features. For example, the feature is preferentially the first frequency, the first frequency energy, the second frequency, the second frequency energy, the third frequency, the third frequency energy, the fourth frequency, the fourth frequency energy, the fifth frequency, the fifth frequency energy, and normalizes the extracted energies. Then, the doctor selects the desired frequency. At this time, the apparatus 100 for analyzing an ECG signal displays the intracardiac waveform corresponding to the collection point in all collection points having the selected frequency on the three-dimensional construction diagram of the left atrium according to the energy level. For example, when the frequency selected by the doctor is 6.4 Hz, all the collected points, as long as the intracardiac waveform with the frequency of 6.4 Hz appears, are displayed on the display interface of the three-dimensional mapping system according to the energy level, so that the doctor can conveniently comprehend the distribution of different frequencies in the heart, which is helpful to make accurate determination. Further, the output module of the apparatus 100 for analyzing an ECG signal outputs the prompt information to the display interface, and displays the possible focal region on the three-dimensional heart model by different colors or shades (represented by different filling patterns in FIG 22).

In this embodiment, by embedding the apparatus 100 for analyzing an ECG signal into the three-dimensional mapping system, the spectrogram analysis function in the three-dimensional mapping system can be added.

In an embodiment, a computer device is provided, including a processor and a memory storing a computer program; when the processor executes the computer program, the following steps are implemented: acquiring a first intracardiac electrical signal in a region of interest or at a reference point/in a reference region to obtain a first intracardiac waveform; generating a first comparison signal spectrogram according to the first intracardiac waveform; acquiring a second intracardiac electrical signal at a point of interest to acquire a second intracardiac waveform; generating a second comparison signal spectrogram according to the second intracardiac waveform; performing a comparative analysis on the first comparison signal spectrogram and the second comparison signal spectrogram and generating a comparative analysis result; and outputting prompt information indicating whether the point of interest is a focal point according to the comparative analysis result.

Those of ordinary skill in the art may understand that all or part of the processes in the method of the above embodiments may be completed by instructing relevant hardware through a computer program, and the computer program may be stored in a non-transitory computer readable storage medium, when the computer program is executed, the process of the foregoing method embodiments may be included. Any reference to the memory, storage, database or other media used in the embodiments provided in this disclosure may include non-transitory and / or transitory memory. Non-transitory memory may include read-only memory (ROM), programmable ROM (PROM), electrically programmable ROM (EPROM), electrically erasable programmable ROM (EEPROM), or flash memory. Volatile memory can include random access memory (RAM) or external cache memory. By way of illustration and not limitation, RAM is available in many forms, such as static RAM (SRAM), dynamic RAM (DRAM), synchronous DRAM (SDRAM), double data rate SDRAM (DDRSDRAM), enhanced SDRAM (ESDRAM), synchronous chain (Synch link) DRAM (SLDRAM), memory bus (Rambus) direct RAM (RDRAM), direct memory bus dynamic RAM (DRDRAM), and memory bus dynamic RAM (RDRAM), etc.

The technical features of the above embodiments can be arbitrarily combined. To simplify the description, all possible combinations of the technical features in the above embodiments are not described. However, as long as there is no contradiction in the combination of these technical features, all should be considered to be in the scope of this disclosure.

The above-mentioned embodiments are merely several exemplary embodiments of the present disclosure, and their descriptions are more specific and detailed, but they should not be understood as limiting the scope of the disclosure. It should be noted that, those of ordinary skill in the art can make a number of modifications and improvements without departing from the concept of the present disclosure, which all fall within the protection scope of the present disclosure. Therefore, the protection scope of the present disclosure shall be subject to the appended claims.

## Claims

1. An apparatus (100) for analyzing an ECG signal, comprising
a first acquisition module (110), configured to acquire N first intracardiac electrical signals in a region of interest or at a reference point/in a reference region to obtain N first intracardiac waveforms, wherein N is an integer greater than or equal to 1;
a first generation module (120), configured to generate a first comparison signal spectrogram according to the N first intracardiac waveforms;
a second acquisition module (130), configured to acquire M second intracardiac electrical signals at a point of interest to acquire M second intracardiac waveforms, wherein M is an integer greater than or equal to 1;
a second generation module (140), configured to generate a second comparison signal spectrogram according to the M second intracardiac waveforms;
a first analysis module (150), configured to perform a comparative analysis on the first comparison signal spectrogram and the second comparison signal spectrogram, and generate a comparative analysis result; and
an output module (160), configured to output prompt information indicating whether the point of interest is a focal point according to the comparative analysis result;
wherein the first generation module (120) further comprises:
a first processing unit (121), configured to generate a first single-signal spectrogram of each first intracardiac waveform to obtain N first single-signal spectrograms; and
a second processing unit (122), configured to combine the N first single-signal spectrograms to generate the first comparison signal spectrogram, or select one first single-signal spectrogram from the N first single-signal spectrograms as the first comparison signal spectrogram.

2. The apparatus (100) for analyzing the ECG signal according to claim 1, wherein the first processing unit (121) further comprises:
a conversion unit (1211), configured to perform a frequency domain conversion on each first intracardiac waveform to acquire the N first single-signal spectrograms;
a pre-processing unit (1212), configured to pre-process each first intracardiac waveform before performing the frequency domain conversion on each first intracardiac waveform to make each first intracardiac waveform in a preset frequency range; and/or,
a smoothing unit (1213), configured to smooth each first single-signal spectrogram after performing the frequency domain conversion on each first intracardiac waveform.

3. The apparatus (100) for analyzing the ECG signal according to claim 1, wherein the second processing unit (122) further comprises:
an accumulation processing unit (1221), configured to perform accumulation processing on the N first single-signal spectrograms to generate the first comparison signal spectrogram; or
a superimposing unit (1222), configured to superimpose the N first single-signal spectrograms to generate the first comparison signal spectrogram.

4. The apparatus (100) for analyzing the ECG signal according to any one of claims 1 to 3, wherein the first analysis module (150) further comprises:
a similarity analysis unit (151), configured to perform a similarity analysis on the second comparison signal spectrogram and the first comparison signal spectrogram to obtain a similarity value; and a first determination unit, configured to determine a magnitude relationship between the similarity value and a preset similarity threshold;
and/or,
a calculation unit (153), configured to calculate an energy difference between an energy corresponding to each frequency in the second comparison signal spectrogram and an energy corresponding to a same frequency in the first comparison signal spectrogram to acquire a plurality of energy differences; and a second determination unit (154), configured to determine a magnitude relationship between the plurality of energy differences and a preset energy threshold.

5. The apparatus (100) for analyzing the ECG signal according to claim 1, further comprising:
a third acquisition module (170), configured to acquire K third intracardiac electrical signals in the region of interest or at the reference point/in the reference region after the focal point is treated, to obtain K third intracardiac waveforms, wherein K is an integer greater than or equal to 1;
a third generation module (180), configured to generate a third comparison signal spectrogram according to the K third intracardiac waveforms;
a second analysis module (190), configured to perform a comparative analysis on the third comparison signal spectrogram and at least one of the first comparison signal spectrogram and the second comparison signal spectrogram to provide information of a treatment effect of the focal point.

6. A three-dimensional mapping system (2000), comprising the apparatus (100) for analyzing the ECG signal of any one of claims 1 to 5, and a display module (200) configured to display a three-dimensional model of a heart; wherein the output module (160) is configured to output the prompt information to the display module (200) which displays the prompt information on the three-dimensional model of the heart.

## Patentansprüche

1. Einrichtung (100) zum Analysieren eines EKG-Signals, umfassend
ein erstes Erfassungsmodul (110), das so konfiguriert ist, dass es N erste intrakardiale elektrische Signale in einem Bereich von Interesse oder an einem Referenzpunkt/in einem Referenzbereich erfasst, um N erste intrakardiale Wellenformen zu erhalten, wobei N eine ganze Zahl größer oder gleich 1 ist;
ein erstes Erstellungsmodul (120), das so konfiguriert ist, dass es ein erstes Vergleichssignalspektrogramm gemäß den N ersten intrakardialen Wellenformen erstellt;
ein zweites Erfassungsmodul (130), das so konfiguriert ist, dass es M zweite intrakardiale elektrische Signale an einem Punkt von Interesse erfasst, um M zweite intrakardiale Wellenformen zu erfassen, wobei M eine ganze Zahl größer oder gleich 1 ist;
ein zweites Erstellungsmodul (140), das so konfiguriert ist, dass es ein zweites Vergleichssignalspektrogramm gemäß den M zweiten intrakardialen Wellenformen erstellt;
ein erstes Analysemodul (150), das so konfiguriert ist, dass es eine vergleichende Analyse des ersten Vergleichssignalspektrogramms und des zweiten Vergleichssignalspektrogramms durchführt und ein vergleichendes Analyseergebnis generiert; und
ein Ausgabemodul (160), das so konfiguriert ist, dass es zeitnahe Informationen ausgibt, die angeben, ob der interessierende Punkt gemäß dem Ergebnis der vergleichenden Analyse ein Brennpunkt ist;
wobei das erste Erstellungsmodul (120) ferner Folgendes umfasst:
eine erste Verarbeitungseinheit (121), die so konfiguriert ist, dass sie ein erstes Einzelsignalspektrogramm jeder ersten intrakardialen Wellenform erstellt, um N erste Einzelsignalspektrogramme zu erhalten; und
eine zweite Verarbeitungseinheit (122), die so konfiguriert ist, dass sie die N ersten Einzelsignalspektrogramme kombiniert, um das erste Vergleichssignalspektrogramm zu erstellen, oder dass sie eines der N ersten Einzelsignalspektrogramme als erstes Vergleichssignalspektrogramm auswählt.

2. Einrichtung (100) zum Analysieren des EKG-Signals nach Anspruch 1, wobei die erste Verarbeitungseinheit (121) ferner Folgendes umfasst:
eine Konvertierungseinheit (1211), die so konfiguriert ist, dass sie eine Frequenzbereichskonvertierung an jeder ersten intrakardialen Wellenform durchführt, um die ersten N Einzelsignalspektrogramme zu erfassen;
eine Vorverarbeitungseinheit (1212), die so konfiguriert ist, dass sie jede erste intrakardiale Wellenform vorverarbeitet, bevor die Frequenzbereichsumwandlung an jeder ersten intrakardialen Wellenform durchgeführt wird, um jede erste intrakardiale Wellenform in einen voreingestellten Frequenzbereich zu bringen; und/oder
eine Glättungseinheit (1213), die so konfiguriert ist, dass sie jedes erste Einzelsignalspektrogramm glättet, nachdem die Frequenzbereichsumwandlung an jeder ersten intrakardialen Wellenform durchgeführt wurde.

3. Einrichtung (100) zum Analysieren des EKG-Signals nach Anspruch 1, wobei die zweite Verarbeitungseinheit (122) ferner Folgendes umfasst:
eine Akkumulationsverarbeitungseinheit (1221), die so konfiguriert ist, dass sie eine Akkumulationsverarbeitung der ersten N Einzelsignalspektrogramme durchführt, um das erste Vergleichssignalspektrogramm zu erstellen; oder
eine Überlagerungseinheit (1222), die so konfiguriert ist, dass sie die N ersten Einzelsignalspektrogramme überlagert, um das erste Vergleichssignalspektrogramm zu erstellen.

4. Einrichtung (100) zum Analysieren des EKG-Signals nach einem der Ansprüche 1 bis 3, wobei das erste Analysemodul (150) ferner Folgendes umfasst:
eine Ähnlichkeitsanalyseeinheit (151), die so konfiguriert ist, dass sie eine Ähnlichkeitsanalyse des zweiten Vergleichssignalspektrogramms und des ersten Vergleichssignalspektrogramms durchführt, um einen Ähnlichkeitswert zu erhalten; und eine erste Bestimmungseinheit, die so konfiguriert ist, dass sie eine Größenbeziehung zwischen dem Ähnlichkeitswert und einem voreingestellten Ähnlichkeitsschwellenwert ermittelt;
und/oder
eine Berechnungseinheit (153), die so konfiguriert ist, dass sie eine Energiedifferenz zwischen einer Energie, die jeder Frequenz im zweiten Vergleichssignalspektrogramm entspricht, und einer Energie, die einer selben Frequenz im ersten Vergleichssignalspektrogramm entspricht, berechnet, um eine Vielzahl von Energiedifferenzen zu erfassen; und eine zweite Bestimmungseinheit (154), die so konfiguriert ist, dass sie eine Größenbeziehung zwischen der Vielzahl von Energiedifferenzen und einem voreingestellten Energieschwellenwert bestimmt.

5. Einrichtung (100) zum Analysieren des EKG-Signals nach Anspruch 1, ferner umfassend:
ein drittes Erfassungsmodul (170), das so konfiguriert ist, dass es K dritte intrakardiale elektrische Signale im Bereich von Interesse oder am Referenzpunkt/im Referenzbereich nach der Behandlung des Fokuspunktes erfasst, um K dritte intrakardiale Wellenformen zu erhalten, wobei K eine ganze Zahl größer oder gleich 1 ist;
ein drittes Erstellungsmodul (180), das so konfiguriert ist, dass es ein drittes Vergleichssignalspektrogramm gemäß den K dritten intrakardialen Wellenformen erstellt;
ein zweites Analysemodul (190), das so konfiguriert ist, dass es eine vergleichende Analyse des dritten Vergleichssignalspektrogramms und mindestens eines des ersten Vergleichssignalspektrogramms und zweiten Vergleichssignalspektrogramms durchführt, um Informationen über einen Behandlungseffekt des Brennpunktes bereitzustellen.

6. Dreidimensionales Kartierungssystem (2000), umfassend die Einrichtung (100) zum Analysieren des EKG-Signals nach einem der Ansprüche 1 bis 5 und ein Anzeigemodul (200), das so konfiguriert ist, dass es ein dreidimensionales Modell eines Herzens anzeigt; wobei das Ausgabemodul (160) so konfiguriert ist, dass die zeitnahen Informationen an das Anzeigemodul (200) ausgibt, das die zeitnahen Informationen auf dem dreidimensionalen Modell des Herzens anzeigt.

## Revendications

1. Appareil (100) d'analyse d'un signal ECG, comprenant
un premier module d'acquisition (110), configuré pour acquérir N premiers signaux électriques intracardiaques dans une région d'intérêt ou en un point de référence/dans une région de référence pour obtenir N premières formes d'onde intracardiaques, dans lequel N est un entier supérieur ou égal à 1 ;
un premier module de génération (120), configuré pour générer un premier spectrogramme de signal de comparaison selon les N premières formes d'onde intracardiaques ;
un deuxième module d'acquisition (130), configuré pour acquérir M deuxièmes signaux électriques intracardiaques en un point d'intérêt afin d'acquérir M deuxièmes formes d'onde intracardiaques, dans lequel M est un entier supérieur ou égal à 1;
un deuxième module de génération (140), configuré pour générer un deuxième spectrogramme de signal de comparaison selon les M deuxièmes formes d'onde intracardiaques ;
un premier module d'analyse (150), configuré pour effectuer une analyse comparative sur le premier spectrogramme du signal de comparaison et le deuxième spectrogramme du signal de comparaison, et générer un résultat d'analyse comparative ; et
un module de sortie (160), configuré pour fournir des informations d'invite indiquant si le point d'intérêt est un point focal selon le résultat de l'analyse comparative ;
dans lequel le premier module de génération (120) comprend en outre :
une première unité de traitement (121), configurée pour générer un premier spectrogramme de signal unique de chaque première forme d'onde intracardiaque afin d'obtenir N premiers spectrogrammes de signal unique ; et
une deuxième unité de traitement (122), configurée pour combiner les N premiers spectrogrammes de signal unique afin de générer le premier spectrogramme de signal de comparaison, ou pour sélectionner un premier spectrogramme de signal unique parmi les N premiers spectrogrammes de signal unique comme premier spectrogramme de signal de comparaison.

2. Appareil (100) d'analyse du signal ECG selon la revendication 1, dans lequel la première unité de traitement (121) comprend en outre :
une unité de conversion (1211), configurée pour effectuer une conversion dans le domaine fréquentiel sur chaque première forme d'onde intracardiaque afin d'acquérir les N premiers spectrogrammes de signal unique ;
une unité de prétraitement (1212), configurée pour prétraiter chaque première forme d'onde intracardiaque avant d'effectuer la conversion dans le domaine fréquentiel sur chaque première forme d'onde intracardiaque afin de faire en sorte que chaque première forme d'onde intracardiaque se trouve dans une plage de fréquences prédéfinie ; et/ou,
une unité de lissage (1213), configurée pour lisser chaque premier spectrogramme de signal unique après avoir effectué la conversion dans le domaine fréquentiel sur chaque première forme d'onde intracardiaque.

3. Appareil (100) d'analyse du signal ECG selon la revendication 1, dans lequel la deuxième unité de traitement (122) comprend en outre :
une unité de traitement d'accumulation (1221), configurée pour effectuer un traitement d'accumulation sur les N premiers spectrogrammes de signal unique afin de générer le premier spectrogramme de signal de comparaison ; ou
une unité de superposition (1222), configurée pour superposer les N premiers spectrogrammes de signal unique afin de générer le premier spectrogramme de signal de comparaison.

4. Appareil (100) d'analyse du signal ECG selon l'une quelconque des revendications 1 à 3, dans lequel le premier module d'analyse (150) comprend en outre :
une unité d'analyse de similarité (151), configurée pour effectuer une analyse de similarité sur le deuxième spectrogramme du signal de comparaison et le premier spectrogramme du signal de comparaison afin d'obtenir une valeur de similarité ; et une première unité de détermination, configurée pour déterminer une relation d'amplitude entre la valeur de similarité et un seuil de similarité prédéfini ;
et/ou,
une unité de calcul (153), configurée pour calculer une différence d'énergie entre une énergie correspondant à chaque fréquence dans le deuxième spectrogramme du signal de comparaison et une énergie correspondant à une même fréquence dans le premier spectrogramme du signal de comparaison afin d'acquérir une pluralité de différences d'énergie ; et une deuxième unité de détermination (154), configurée pour déterminer une relation d'amplitude entre la pluralité de différences d'énergie et un seuil d'énergie prédéfini.

5. Appareil (100) d'analyse du signal ECG selon la revendication 1, comprenant en outre :
un troisième module d'acquisition (170), configuré pour acquérir K troisièmes signaux électriques intracardiaques dans la région d'intérêt ou au niveau du point de référence/dans la région de référence après le traitement du point focal, afin d'obtenir K troisièmes formes d'onde intracardiaques, dans lequel K est un entier supérieur ou égal à 1 ;
un troisième module de génération (180), configuré pour générer un troisième spectrogramme de signal de comparaison selon les K troisièmes formes d'onde intracardiaques ;
un deuxième module d'analyse (190), configuré pour effectuer une analyse comparative sur le troisième spectrogramme de signal de comparaison et au moins l'un des premier et deuxième spectrogrammes de signal de comparaison afin de fournir des informations sur un effet de traitement du point focal.

6. Système de cartographie tridimensionnelle (2000), comprenant l'appareil (100) pour analyser le signal ECG selon l'une quelconque des revendications 1 à 5, et un module d'affichage (200) configuré pour afficher un modèle tridimensionnel d'un cœur ; dans lequel le module de sortie (160) est configuré pour transmettre les informations d'invite au module d'affichage (200) qui affiche les informations d'invite sur le modèle tridimensionnel du cœur.
